# EUROPÄISCHE PATENTSCHRIFT

(11) **EP 1 308 157 B1**
(45) Veröffentlichungstag und Bekanntmachung des Hinweises auf die Patenterteilung: **05.04.2006**
(21) Anmeldenummer: 02022978.7
(22) Anmeldetag: 14.10.2002
(51) Int. Cl.: A61K 31/00, C07F 15/04, C07F 15/00

(54) **Komplexe N-heterocyclischer Carbene und ihre Verwendung**
N-heterocyclic carbene complexes and the use thereof
Complexes de carbènes N-heterocycliques et leur utilisation

(30) Priorität: 26.10.2001 DE 10152989
(43) Veröffentlichungstag der Anmeldung: 07.05.2003
(73) Patentinhaber: LANXESS Deutschland GmbH, 51369 Leverkusen (DE)
(72) Erfinder: Militzer, Hans-Christian, Dr., 51519 Odenthal (DE); Scholz, Ulrich, Dr., 45475 Mülheim (DE); Herrmann, Wolfgang A., Prof. Dr., 85354 Freising (DE); Gstöttmayr, Christian, 4055 Basel (CH)

(56) Entgegenhaltungen:
- WO-A-01/16057
- WO-A-01/66248
- WO-A-98/00399
- FR-A- 2 801 887
- BOEHM V P W ET AL: "Nickel-catalyzed cross-coupling of aryl chlorides with aryl grignard reagents" ANGEWANDTE CHEMIE. INTERNATIONAL EDITION, VERLAG CHEMIE. WEINHEIM, DE, Bd. 39, Nr. 9, 2. Mai 2000 (2000-05-02), Seiten 1602-1604, XP002178608 ISSN: 0570-0833
- BOHM V P W ET AL: "N-Heterocyclic carbenes - Part 26. N-Heterocyclic carbene complexes of palladium(0): synthesis and application in the Suzuki cross-coupling reaction" JOURNAL OF ORGANOMETALLIC CHEMISTRY, ELSEVIER-SEQUOIA S.A. LAUSANNE, CH, Bd. 595, Nr. 2, Februar 2000 (2000-02), Seiten 186-190, XP004187387 ISSN: 0022-328X
- HERRMANN W A ET AL: "N-Heterocyclic Carbenes" ANGEWANDTE CHEMIE. INTERNATIONAL EDITION, VERLAG CHEMIE. WEINHEIM, DE, Bd. 36, 1997, Seiten 2162-2187, XP002178612 ISSN: 0570-0833
- HERMANN W A ET AL: "Synthesis, structure and catalytic aplication of Pd(II)complexes bearing N-heterocyclic carbenes and phosphines" JOURNAL OF ORGANOMETALLIC CHEMISTRY, ELSEVIER-SEQUOIA S.A. LAUSANNE, CH, Bd. 617-618, 15. Januar 2001 (2001-01-15), Seiten 616-628, XP002178611 ISSN: 0022-328X
- A ET AL: "Chelating N-heterocyclic carbene ligands in palladium-catalyzed heck-type reactions" JOURNAL OF ORGANOMETALLIC CHEMISTRY, ELSEVIER-SEQUOIA S.A. LAUSANNE, CH, Bd. 557, Nr. 1, 20. April 1998 (1998-04-20), Seiten 93-96, XP004122368 ISSN: 0022-328X
- CADDICK S ET AL: "An improved synthesis of bis(1,3-di-N-tert-butylimidazol-2-ylidene) pa lladium(0) and its use in C-C and C-N coupling reactions" JOURNAL OF ORGANOMETALLIC CHEMISTRY, ELSEVIER-SEQUOIA S.A. LAUSANNE, CH, Bd. 617-618, 15. Januar 2001 (2001-01-15), Seiten 635-639, XP004315086 ISSN: 0022-328X
- BAKER M V ET AL: "Palladium carbene complexes derived from imidazolium-linked ortho-cyclophanes" JOURNAL OF THE CHEMICAL SOCIETY, DALTON TRANSACTIONS, CHEMICAL SOCIETY. LETCHWORTH, GB, 21. Januar 2001 (2001-01-21), Seiten 111-120, XP002178609 ISSN: 1472-7773
- "Neue Wege zu N-heterocyclischen Carbenen und deren Metallkomplexen: Anwendungen in der Homogenkatalyse" DISSERTATION FAKULTAET FUER CHEMIE, BIOLOGIE UND GEOWISSENSCHAFTEN DER TECHNISCHEN UNIVERSITAET MUENCHEN ZUR ERLANGUNG DES AKADEMISCHEN GRADES EINES DOKTORS DER NATURWISSENSCHAFTEN, XX, XX, 1997, Seiten 30-59,124-172, XP002212740

## Beschreibung

Die vorliegende Erfindung betrifft Komplexe von N-heterocyclischen Carbenen, deren Verwendung sowie die Vorstufen solcher Komplexe.

Polyarylverbindungen, insbesondere substituierte Biphenyle, sind von großer Bedeutung als Feinchemikalien und Zwischenprodukte für die Herstellung von Arzneimitteln und Agrochemikalien.

Die Synthese von Polyarylverbindungen kann beispielsweise durch Übergangsmetall-katalysierte Kupplung von aktivierten Brom- oder Iodaromaten mit Arylgrignard-, Arylzink-, Trialkylaryl-Verbindungen oder Arylboronsäuren gegebenenfalls in Gegenwart einer Base erfolgen (siehe z. B. Suzuki, A. J., J. Organomet. Chem., 576, 1999, 329-340). Der Nachteil der im Zitat beschriebenen Synthese unter Verwendung von Arylboronsäuren ist jedoch die Verwendung der teuren Brom- und Iodaromaten sowie die zum Teil sehr drastischen Reaktionsbedingungen.

Für die Herstellung von Polyarylverbindungen aus den preiswerteren Chloraromaten wurden schon Übergangsmetall-Komplexe N-heterocyclischer Carbene als Katalysatoren getestet. Zhang und Trudell entwickelten beispielsweise (Tetrahedron Letters, 41, 2000, S. 595-598) ein Verfahren bei dem Bis-imidazol-2-yliden-Palladium-Komplexe als Katalysatoren eingesetzt werden, die in situ aus Palladiumacetat und Bis-imidazolium-Salzen erzeugt werden. Die Herstellung solcher Bis-imidazolium-Salze ist jedoch sehr aufwändig und das Verfahren daher für den großtechnischen Einsatz nicht geeignet.

Ein ähnliches Verfahren von Nolan (J. Org. Chem., 1999, 64, 3804-3805) basiert auf der Katalyse durch in situ erzeugte Systeme von Palladium-dibenzylidenaceton [Pd₂(dba)₃] und dem Mono-imidazolium-Salz 1,3-Bis-(2,4,6-trimethylphenyl)imidazolium-hydrochlorid. Die Reaktivität dieser Systeme ist jedoch sehr stark von der Wahl der eingesetzten Base abhängig und ist wie Böhm et al. (J. Organomet. Chem., 595, 2000, S. 186-190) zeigen konnten, nicht auf andere Mono-imidazolium-Salze übertragbar. So ist beispielsweise das System Palladium-dibenzylidenaceton [Pd₂(dba)₃] und 1,3-Bis-(tert.-butyl)imidazolium-tetrafluoroborat katalytisch völlig inaktiv.

Die gleichen Autoren setzten daher als Katalysatoren auch definierte, isolierbare Palladium-Imidazol-2-yliden-Komplexe des Typs [Pd(Imidazol-2-yliden)₂] ein. Die verwendeten, isolierten Komplexe zeigten jedoch nur mäßige Umsatzraten bei 80°C. Bei 40°C wurde keine Umsetzung beobachtet.

WO9800399 beschreibt ein Verfahren zur Herstellung von ungesättigten stickstoffhaltigen Verbindungen, wobei Verbindungen mit -NH- oder -NH₂- funktionellen Gruppen mit Alkyl- oder Arylhalogeniden in Gegenwart stöchiometrischer Mengen einer starken Base und einer Mischung enthaltend Katalysatorvorstufen umgesetzt werden. Die Mischung enthaltend Katalysatorvorstufen enthält nullwertiges Nickel und Phosphan - oder Carbenliganden, wobei letztere auch 1,3 -Diadamantylimidazolin-2-yliden-Liganden sein können.

Es bestand daher das Bedürfnis, Katalysatoren zu entwickeln, die sich in vorteilhafter Weise für ein Verfahren zur Herstellung von Polyarylverbindungen, insbesondere ausgehend von Arylchloriden, unter milden Reaktionsbedingungen eignen.

Es wurde nun ein Verfahren zur Herstellung von Polyarylverbindungen gefunden, das dadurch gekennzeichnet ist, dass
- Arylhalogenide oder Arylsulfonate
- mit reaktiven Arylverbindungen
- gegebenenfalls in Gegenwart von Base und
- gegebenenfalls in Gegenwart von Lösungsmittel und
- in Gegenwart eines Katalysators, der zumindest einen Komplex eines Übergangsmetalls ausgewählt aus der Gruppe Nickel, Palladium oder Platin enthält, der wiederum als Ligand mindestens ein N-heterocyclisches Carben der allgemeinen Formel (I) enthält, 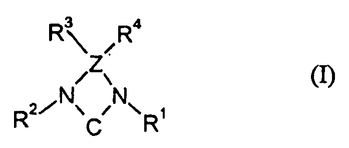
in der
- Z: für einen 1,2-Ethandiyl- oder 1,2-Ethendiyl-Rest, bevorzugt einen 1,2-Ethendiyl-Rest, steht und
- R¹ und R²: jeweils unabhängig voneinander für Reste der allgemeinen Formel (II)

CR⁵R⁶R⁷ (II)

stehen, in der
a) CR⁵R⁶R⁷ zusammen für einen substituierten oder unsubstituierten carbocyclischen oder heterocyclischen Rest oder einen substituierten oder unsubstituierten carbopolycyclischen oder heteropolycyclischen Rest steht oder
b) die Reste R⁵, R⁶ und R⁷ jeweils für Wasserstoff oder einen organischen Rest stehen,
   wobei sowohl für a) als auch b) die Auflage gilt, dass von denjenigen drei Atomen von R⁵, R⁶ und R⁷, die an das Kohlenstoffatom C gebunden sind, entweder
   - alle drei jeweils unabhängig voneinander sekundäre, tertiäre oder quartäre Kohlenstoffatome sind oder
   - zwei jeweils unabhängig voneinander sekundäre, tertiäre oder quartäre Kohlenstoffatome sind und für den Fall, dass beide dieser zwei Atome sekundär sind, zumindest eines davon an insgesamt mindestens zwei tertiäre oder quartäre Kohlenstoffatome gebunden ist, und
- R³ und R⁴: jeweils unabhängig voneinander für
Wasserstoff, C₆-C₁₂-Aryl, wie z.B. Phenyl, C₆-C₁₂-Arylalkyl wie z.B. Benzyl oder C₁-C₈-Alkyl wie z.B. Methyl, Ethyl oder Isospropyl steht,
miteinander umgesetzt werden.

Reaktive Arylverbindungen sind beispielsweise und bevorzugt Trialkylzinnaryl-Verbindungen, Arylboronsäuren, Arylzink- und Arylmagnesiumverbindungen.

Bevorzugt werden im erfindungsgemäßen Verfahren zur Herstellung von Polyarylverbindungen Arylhalogenide oder Arylsulfonate der allgemeinen Formel (III) und Arylverbindungen der allgemeinen Formeln (IVa, b, c und d), eingesetzt, von denen Arylverbindungen der Formel (IVa) noch weiter bevorzugt sind.

In den Formeln

Ar¹-Y (III)

Ar²-B(OH)₂ (IVa)

Ar²-Sn(C₁-C₆-Alkyl)₃ (IVb)

Ar²-ZnX (IVc)

Ar²-MgX (IVd)

stehen
- Ar¹ und Ar²: jeweils unabhängig voneinander für einen substituierten oder unsubstituierten aromatischen Rest oder für einen substituierten oder unsubstituierten heteroaromatischen Rest und
- Y: für Chlor, Brom, Iod oder ein Sulfonat und
- X: für Chlor, Brom oder Iod.

Sulfonate sind beispielsweise Trifluormethansulfonat, Pentafluorethansulfonat oder Nonafluorbutansulfonat.

Besonders bevorzugt steht Y für Chlor oder Brom, ganz besonders bevorzugt für Chlor.

Substituierte oder unsubstituierte aromatische Reste sind bevorzugt carbocyclische aromatische Reste mit 6 bis 24 Gerüstkohlenstoffatomen wie z.B. Phenyl, Naphtyl, Biphenyl, Binaphtyl oder Anthracenyl, die weiterhin mit bis zu fünf gleichen oder verschiedenen Substituenten pro Cyclus substituiert sein können.

Substituierte oder unsubstituierte heteroaromatische Reste sind bevorzugt heteroaromatische Reste mit 5 bis 24 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können wie z.B. Pyrrol, Pyrazolyl, Pyrimidinyl, Pyridinyl, Oxazolyl, Thiophen-yl, Furanyl, Indolyl, Triazolyl, Thiazolyl, Dibenzofuranyl, Dibenzothiophen-yl oder Chinolinyl und die weiterhin mit bis zu fünf gleichen oder verschiedenen Substituenten pro Cyclus substituiert sein können.

Substituenten für carbocyclische aromatische oder heteroaromatische Reste können beispielsweise ausgewählt sein aus der Gruppe OH, Iod, Brom, Chlor, Fluor, Nitro, Cyano, freies oder geschütztes Formyl, C₁-C₁₂-Alkyl, C₂-C₁₂-Alkenyl, C₆-C₁₂-Aryl, C₇-C₁₃-Arylalkyl, C₁-C₈-Hydroxyalkyl, C₁-C₈-Hydroxyalkoxy, C₁-C₈-Hydroxyalkylamino, -PO-[(C₁-C₈)-Alkyl]₂, -PO-[(C₆-C₁₂)-Aryl]₂, Tri(C₁-C₆-alkyl)siloxyl oder Resten der allgemeinen Formel (V),

A-B-D-E (V)

in der unabhängig voneinander
- A: fehlt, für einen C₁-C₈-Alkylenrest oder einen C₂-C₈-Alkenylenrest steht und
- B: fehlt oder für Sauerstoff, Schwefel oder NR⁸ steht,
wobei R⁸ Wasserstoff, C₁-C₈-Alkyl, C₇-C₁₀-Arylalkyl oder C₆-C₁₀-Aryl bedeutet und
- D: für eine Carbonyl-Gruppe steht und
- E: für R⁹, OR⁹ oder N(R¹⁰)₂ steht,
wobei
R⁹ für Wasserstoff, C₁-C₈-Alkyl, C₇-C₁₀-Arylalkyl, C₁-C₈-Hydroxyalkyl, C₁-C₈-Halogenalkyl oder C₆-C₁₀-Aryl und
R¹⁰ jeweils unabhängig für Wasserstoff, C₁-C₈-Alkyl, C₁-C₈-Hydroxyalkyl, C₇-C₁₀-Arylalkyl oder C₆-C₁₀-Aryl oder N(R¹⁰)₂ zusammen für einen cyclischen Aminorest steht,
oder Resten der allgemeinen Formeln (VIa-e)

A-E (VIa)

A-SO₂-E (VIb)

A-B-SO₂R⁹ (VIc)

A-SO₃X (VId)

A-COX (VIe)

in denen A, B, E und R⁹ die oben angegebene Bedeutung besitzen und X für OM steht, wobei M ein Alkalimetallion, ein halbes Äquivalent eines Erdalkalimetallions, ein Ammoniumion oder ein organisches Ammoniumion bedeuten kann. M steht bevorzugt für Lithium, Natrium, Kalium, Ammonium oder organische Ammoniumionen der allgemeinen Formel (VII)

[NHₙ(C₁-C₁₂-Alkyl)ₘ(C₂-C₆-Hydroxyalkyl)ₚ(C₇-C₁₂-Arylalkyl)_{q}(C₆-C₁₀-Aryl)ᵣ]⁺ (VII),

in der (n+m+p+q+r) = 4 ergibt.

M kann auch für Wasserstoff stehen. Im Reaktionsmedium liegen die Säuregruppen dann jedoch in Form der Salze der eingesetzten Base vor.

Alkyl beziehungsweise Alkylen bedeutet in allen Zusammenhängen der Erfindung jeweils unabhängig einen geradkettigen, cyclischen, verzweigten oder unverzweigten Alkyl- beziehungsweise Alkylen-Rest, der gegebenenfalls durch Alkoxy-Gruppen weiter substituiert sein kann. Gleiches gilt für den Alkylteil eines Arylalkyl-Restes.

Die allgemeine Bezeichnung Aryl als Substituent bedeutet in allen Zusammenhängen der Erfindung nicht nur carbocyclische Reste, sondern auch heteroaromatische Reste, in denen keines, ein, zwei oder drei Gerüstkohlenstoffatome pro Cyclus, im gesamten Rest mindestens jedoch ein Gerüstkohlenstoffatom, durch Heteroatome, ausgewählt aus der Gruppe Stickstoff, Schwefel oder Sauerstoff, substituiert sind.

Alkoxy bedeutet in allen Zusammenhängen der Erfindung jeweils unabhängig einen geradkettigen, cyclischen, verzweigten oder unverzweigten Alkoxy-Rest.

Halogenalkyl und Halogenalkoxy bedeuten in allen Zusammenhängen der Erfindung jeweils unabhängig geradkettige, cyclische, verzweigte oder unverzweigte Alkylreste und Alkoxyreste, die mit einem, mehreren oder vollständig mit Fluor- oder Chloratomen substituiert sein können. Weiterhin können diese Reste weiter durch Alkoxyreste substituiert sein.

Besonders bevorzugt stehen Ar¹ und Ar² jeweils unabhängig voneinander für carbocyclische Aryl-Reste Phenyl, Naphtyl, Biphenyl, Binaphtyl oder Anthracenyl oder Heteroaryl-Reste ausgewählt aus der Gruppe Pyrrolyl, Pyrimidinyl, Pyridinyl, Oxazolyl, Thiophen-yl, Furanyl, Indolyl, oder Chinolinyl, die mit keinem, einem, zwei oder drei Substituenten ausgewählt aus der Gruppe OH, Iod, Brom, Chlor, Fluor, Nitro, Cyano, freies oder geschütztes Formyl, C₁-C₄-Alkyl, Benzyl, C₁-C₄-Hydroxyalkyl, -PO-[(C₁-C₈)-Alkyl]₂, -PO-[(C₆-C₁₂-Aryl]₂, oder Resten der allgemeinen Formel (V), in der unabhängig voneinander
- A: fehlt und
- B: fehlt oder für NR⁸ steht,
wobei
R⁸ Wasserstoff oder C₁-C₄-Alkyl bedeutet und
- D: für eine Carbonyl-Gruppe steht und
- E: für R⁹, OR⁹ oder N(R¹⁰)₂ steht,
wobei R⁹ für Wasserstoff, C₁-C₈-Alkyl oder C₆-C₁₀-Aryl und
R¹⁰ jeweils unabhängig für Wasserstoff, C₁-C₈-Alkyl oder N(R¹⁰)₂ zusammen für einen Pyrrolidinyl- oder Morpholinyl- Rest steht,
oder Resten der allgemeinen Formeln (VId) oder (VIe), in denen
A die oben angegebene Bedeutung besitzt und X für ONa oder OK steht, substituiert sind.

Besonders bevorzugt stehen Ar¹ und Ar² jeweils unabhängig voneinander für Phenyl-, Pyrrolyl-, Pyrimidinyl-, Pyridinyl-Reste, die mit keinem, einem oder zwei Substituenten ausgewählt aus der Gruppe Fluor, Nitro, Cyano, Formyl, Methyl, Ethyl, Methoxy, Trifluormethyl, Amino, Dimethylamino, Aminoacetyl, Acetyl, COONa oder SO₃Na weiter substituiert sind.

Ganz besonders bevorzugt werden für das erfindungsgemäße Verfahren als Verbindungen der allgemeinen Formel (III) 4-Chlortoluol, 2-Chlortoluol, 1-Chlor-4-trifluormethylbenzol, 1-Chlor-4-methoxybenzol und 1-Chlor-4-acetylbenzol eingesetzt.

Ganz besonders bevorzugt werden für das erfindungsgemäße Verfahren Verbindungen der allgemeinen Formel (IVa) eingesetzt, besonders bevorzugt Phenylboronsäure, 2-Methylphenylboronsäure und 3-Methoxyphenylboronsäure.

Die in der Reaktion einsetzbaren Arylhalogenide oder Arylsulfonate sowie die reaktiven Arylverbindungen sind entweder kommerziell verfügbar oder nach Literaturvorschriften oder analog dazu herstellbar.

Das molare Verhältnis von Arylhalogenid oder Arylsulfonat zu eingesetzter reaktiver Arylverbindung kann beispielsweise 0,01:1 bis 100:1 betragen, bevorzugt ist ein molares Verhältnis von 0,5:1 bis 5:1, besonders bevorzugt 0,8:1 bis 1: 1,5.

In einer bevorzugten Ausführungsform des erfindungsgemäßen Verfahrens werden als reaktive Arylverbindungen Arylboronsäuren eingesetzt und Basen eingesetzt wie zum Beispiel:
Stickstoffbasen wie beispielsweise Pyridin oder Amine wie Diethylamin oder Triethylamin,
Alkalimetall- oder Erdalkalimetall-hydrogencarbonate, -carbonate, -alkoholate, -carboxylate oder -fluoride oder organische Ammoniumfluoride oder Mischungen von Basen.

Bevorzugt werden die Hydrogencarbonate, Carbonate, (2 Basenäquivalente) Methanolate, Ethanolate, Isopropylate, tert.-Butanolate und Acetate von Lithium, Natrium, Kalium und Cäsium sowie Cäsiumfluorid eingesetzt.

Besonders bevorzugt sind Cäsiumfluorid und -carbonat.

Ganz besonders bevorzugt ist Cäsiumfluorid.

Das molare Verhältnis von Basenäquivalente zu umsetzbaren Arylhalogenid oder Arylsulfonat kann beispielsweise 0,5:1 bis 100:1 betragen, bevorzugt ist ein molares Verhältnis von 1:1 1 bis 5:1, besonders bevorzugt 1:1 1 bis 1,5:1.

Umsetzbar heißt in diesem Zusammenhang derjenige Anteil an Arylhalogenid oder Arylsulfonat, für den ein Äquivalent Arylboronsäure in der Reaktion eingesetzt wird.

Gegebenenfalls wird das erfindungsgemäße Verfahren in Gegenwart eines oder mehrerer aprotischer Lösungsmittel durchgeführt. Bevorzugt sind dies:
Cyclische oder acyclische Ether wie beispielsweise 1,4-Dioxan, Tetrahydrofuran, Diethylether, Methyl-tert.-butylether oder Di-n-butylether, aromatische Kohlenwasserstoffe wie zum Beispiel Toluol, o-Xylol, m-Xylol oder p-Xylol, dipolar aprotische Verbindungen wie zum Beispiel Dimethylformamid oder N-Methylpyrrolidon, Dimethylacetamid, Dimethylsulfoxid oder Mischungen solcher Lösungsmittel, die auch Wasser enthalten können. Bei flüssigen Arylhalogeniden oder Arylsulfonaten kann dieses im Überschuss auch selbst als Lösungsmittel eingesetzt werden.

Besonders bevorzugt ist 1,4-Dioxan.

Die Menge des gegebenenfalls eingesetzten aprotischen Lösungsmittels kann beispielsweise 50 ml bis 5.000 ml, bevorzugt 500 bis 3.000 ml pro Mol des eingesetzten Arylhalogenids oder Arylsulfonats betragen.

Bevorzugte N-heterocyclische Carbene sind solche der allgemeinen Formel (I) in der
- Z: für einen 1,2-Ethendiyl-Rest und die beiden Reste R¹ und R² identisch sind und für Reste der allgemeinen Formel (II) stehen und worin CR⁵R⁶R⁷
- a): jeweils zusammen für einen substituierten oder unsubstituierten C₅-C₂₀ carbocyclischen Rest oder einen substituierten oder unsubstituierten C₆-C₂₄ carbopolycyclischen Rest steht oder
- b): R⁵, R⁶und R⁷ jeweils unabhängig für einen organischen Rest stehen kann der ausgewählt ist aus der Gruppe C₁-C₂₀-Alkyl, C₁-C₁₂-Halogenalkyl, C₇-C₂₀₋Arylalkyl,C₅-C₁₈-Aryl oder Resten der allgemeinen Formel (V)
wobei sowohl für a) als auch b) die oben genannte Auflage gilt.

Besonders bevorzugt sind N-heterocyclische Carbene der allgemeinen Formel (I), in der Z für einen 1,2-Ethendiyl-Rest und die beiden Reste R¹ und R² identisch sind und für Reste der der allgemeinen Formel (II) stehen und worin CR⁵R⁶R⁷
a) jeweils zusammen für jeweils einen der 8 isomeren Menthyl-Reste oder einen substituierten oder unsubstituierten Adamantyl-Rest steht oder
b) R⁵, R⁶ und R⁷ jeweils für Ethyl, n-Propyl, iso-Propyl, n-Butyl, n-Pentyl, n-Hexyl, cyclo-Hexyl, n-Octyl, iso-Octyl, Trifluormethyl, Benzyl, Phenyl, 1-Naphthyl oder 2-Naphthyl stehen.

Substituenten am Adamantyl-Rest können beispielsweise sein:

Oxo, C₁-C₆-Alkoxy, Fluor, (C₁-C₄)-Acyloxy, Cyano, unverzweigtes oder verzweigtes geradkettiges oder cyclisches C₁-C₆-Alkyl wie z.B. Methyl, Ethyl, Isopropyl, Cyclopentyl, Cyclohexyl, (C₁-C₄)-Acylamino, C₁-C₆-Halogenalkyl, wie z.B. Trifluormethyl, (C₁-C₆)-Alkoxycarbonyl, unsubstituiertes oder substituiertes Phenyl wie z.B. Phenyl, Nitrophenyl, p-, o-, m-Tolyl, p-, o-, m-Anisyl.

Ganz besonders bevorzugte N-heterocyclische Carbene der allgemeinen Formel (I) sind 1,3-Di-(1R, 2S, 5R-(-)menthylimidazolin-2-yliden, 1,3-Di-(1S, 2R, 5S-)-(+)methylimidazolin-2-yliden und 1,3-Diadamantylimidazolin-2-yliden.

Am meisten bevorzugt ist als N-heterocyclischer Carbenligand der allgemeinen Formel (I) 1,3-Diadamantylimidazolin-2-yliden.

Bevorzugt werden für das erfindungsgemäße Verfahren als Katalysatoren Nickel- oder Palladiumkomplexe in der formalen Oxidationsstufe null eingesetzt, die pro Metallatom mindestens ein N-heterocyclisches Carben der allgemeinen Formel (I) enthalten, in der Z, R¹, R², R³ und R⁴ jeweils unabhängig von gegebenenfalls vorhandenen weiteren N-heterocyclischen Carbene der allgemeinen Formel (I) die genannte Bedeutung besitzen.

Besonders bevorzugt werden als Katalysatoren Palladiumkomplexe in der formalen Oxidationsstufe null eingesetzt, die pro Metallatom mindestens eine N-heterocyclisches Carben der allgemeinen Formel (I) enthalten, in der Z, R¹, R², R³ und R⁴ jeweils unabhängig von gegebenenfalls vorhandenen weiteren N-heterocyclischen Carbene der allgemeinen Formel (I) die genannte Bedeutung besitzen.

Ganz besonders bevorzugte Komplexe sind Palladiumkomplexe der allgemeinen Formel (VIII)

[Pd(L)₂] (VIII)

in der die beiden Liganden L jeweils unabhängig voneinander für N-heterocyclische Carbene der allgemeinen Formel (I) stehen, in der in der Z, R¹, R², R³ und R⁴ jeweils unabhängig von einander die dort genannte Bedeutung besitzen.

In Formel (II) sind die beiden Liganden L bevorzugt identisch.

Am meisten bevorzugt werden als Katalysatoren die Komplexe [Bis-(1,3-Diadamantylimidazol-2-yliden)-palladium], [Bis-(1,3-Di-(-)-menthylimidazol-2-yliden)-palladium] und [Bis-(1,3-Di-(-)-menthylimidazol-2-yliden)-palladium] eingesetzt, von denen [Bis-(1,3-Diadamantylimidazol-2-yliden)-palladium] noch weiter bevorzugt ist.

Es sei an dieser Stelle darauf hingewiesen, dass alle Vorzugsbereiche in beliebigen Kombinationen von der Erfindung mit umfasst sind.

Die Herstellung der als Katalysatoren eingesetzten Komplexe kann beispielsweise durch Liganden-Substitutionsreaktionen an einem geeigneten Precursor-Komplex erfolgen.

Palladium-Komplexe der allgemeinen Formel (VIII) können beispielsweise analog zu Cloke (J. Organomet. Chem., 2001, 617-618, 635-639) direkt hergestellt werden, indem man die N-heterocyclischen Carbene der allgemeinen Formel (I) mit Allylpalladium-chlorid-dimer und Natriumdimethylmalonat umsetzt.

Weiterhin können beispielsweise Palladiumkomplexe der allgemeinen Formel (VIII) in vorteilhafter Weise auch dadurch hergestellt werden, dass man Palladiumkomplexe der allgemeinen Formel (IX),

[Pd(P)₂] (IX)

in der P für einen monodentaten Phosphanliganden steht, mit den N-heterocyclischen Carbene der allgemeinen Formel (I) in Gegenwart von Lösungsmittel umsetzt. Bevorzugt wird dabei als Palladiumkomplex der allgemeinen Formel (IX) [Bis-(tri-tert.-butylphosphan)palladium] eingesetzt.

Als Lösungsmittel für die Umsetzung eignen sich dafür beispielsweise Ether wie z.B. Tetrahydrofuran, aliphatische oder aromatische Kohlenwasserstoffe wie z.B. Penten, n-Hexan, Cyclohexan, Toluol.

Besonders bevorzugt wird dabei als Lösungsmittel Hexan eingesetzt.

Die Temperatur kann beispielsweise zwischen -20°C und 80°C betragen, bevorzugt sind 10 bis 50 °C, besonders bevorzugt ist Raumtemperatur.

Durch das beschriebene Verfahren, das von der Erfindung ebenfalls umfasst ist, können Palladiumkomplexe der allgemeinen Formel (VIII) in hohen Ausbeuten erhalten werden.

Werden für Synthesen von erfindungsgemäßen Komplexen N-heterocyclische Carbene der allgemeinen Formel (I) benötigt, können diese in an sich bekannter Weise durch Deprotonierung aus den analogen Salzen der allgemeinen Formel (X) erfolgen, 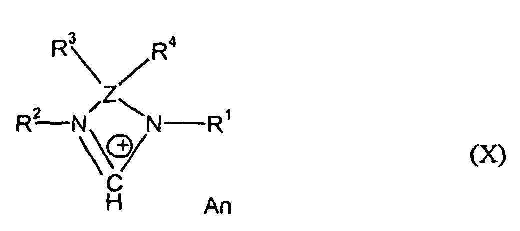 in der Z, R¹, R², R³ und R⁴, die für Formel (I) genannten Bedeutungen besitzen und in der An für das Anion einer Säure steht.

Formel (X) steht stellvertretend für die möglichen tautomeren Verbindungen, die vom Umfang der Erfindung ebenfalls umfasst sind.

Bevorzugt steht An für ein Anion einer Säure, die einen pKa-Wert von 3 oder darunter besitzt. Besonders bevorzugt steht An für Hydrogensulfat, Chlorid, Bromid, Iodid, Tetrafluoroborat, Hexafluorophosphat oder ein halbes Äquivalent Sulfat.

Ganz besonders bevorzugt steht An für Chlorid.

Bevorzugt erfolgt dabei die Deprotonierung durch Alkalimetallhydride wie z.B. Natriumhydrid in einer Mischung aus einem Ether wie z.B. THF und flüssigem Ammoniak bei Temperaturen zwischen -35 und -80°C.

Die erfindungsgemäßen Salze der allgemeinen Formel (X) lassen sich beispielsweise durch stufenweise Alkylierung von Verbindungen der allgemeinen Formel (XI) herstellen 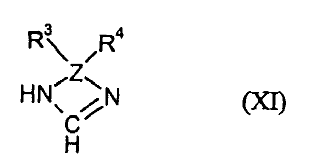 in der
Z, R³ und R⁴ die unter Formel (I) angegebene Bedeutung besitzen.

Sind in den Salzen der allgemeinen Formel (X) die Reste R¹ und R² identisch und steht Z für einen 1,2-Ethendiyl-Rest, erfolgt die Herstellung bevorzugt durch Umsetzung von Aminen der allgemeinen Formel (XII)

H₂N-R¹ (XII)

in der R¹ die unter der allgemeinen Formel (I) genannte Bedeutung besitzt
mit vicinalen Dicarbonylverbindungen,
der allgemeinen Formel (XIII) 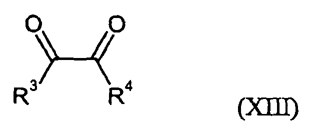 und Formaldehyd
in Gegenwart einer Säure der allgemeinen Formel (XIV)

H-An (XIV).

in der An die unter der allgemeinen Formel (X) angegebene Bedeutung besitzt.

Die Salze der allgemeinen Formel (X) sind ebenfalls Bestandteil der Erfindung und können entweder direkt z.B. nach der Methode von Cloke (loc.cit.) oder nach vorheriger Deprotonierung zur Herstellung der erfmdungsgemäßen Katalysatoren und Komplexe eingesetzt werden.

Die Verbindungen der allgemeinen Formeln (X), in der Z für einen 1,2-Ethandiyl-Rest steht, lassen sich beispielsweise gemäß oder analog zu J. F. Hartwig, Org. Lett. 2000, 2, 10, S. 1423 herstellen.

Die einzelnen Stufen, die zur Herstellung der erfindungsgemäßen Katalysatoren führen können, seien am Beispiel von [Bis-(1,3-Diadamantylimidazol-2-yliden)-palladium] schematisch dargestellt:

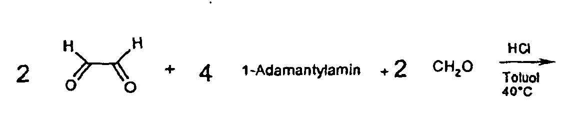

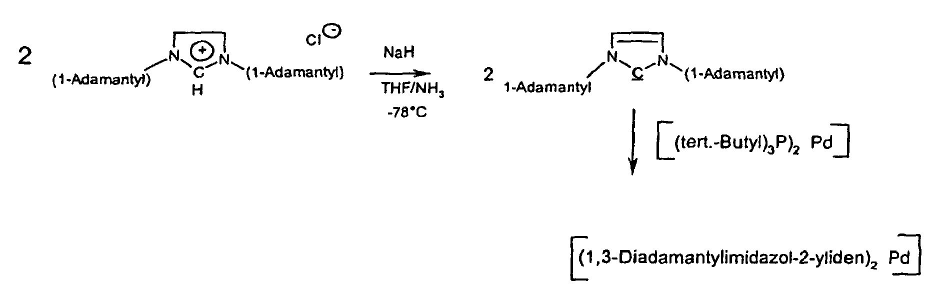

Das erfindungsgemäße Verfahren zur Herstellung von Polyarylverbindungen kann beispielsweise bei einer Reaktionstemperatur von 0 bis 100°C, bevorzugt 20 bis 80°C durchgeführt werden. Ganz besonders bevorzugt ist Raumtemperatur.

Die Reaktionsdauer kann beispielsweise 5 Minuten bis 168 h betragen, bevorzugt sind 20 min bis 25 h.

Die Reaktion kann beispielsweise bei 0,2 bis 100 bar durchgeführt werden, bevorzugt ist Umgebungsdruck.

Die Reaktion wird bevorzugt unter Schutzgas und unter weitgehendem Ausschluss von Sauerstoff und Feuchtigkeit durchgeführt. Als Schutzgase kommen beispielsweise Stickstoff und Edelgase wie beispielsweise Argon oder Mischungen davon in Frage.

In einer bevorzugten Ausführungsform legt man die Arylboronsäure der allgemeinen Formel (IVa), den Komplex der allgemeinen Formel (VIII) und die Base gegebenenfalls in einem Lösungsmittel unter Schutzgasatmosphäre vor und gibt dann das Arylhalogenid oder das Arylsulfonat gegebenfalls gelöst in einem Lösungsmittel zu und lässt bei einer Temperatur zwischen 20 und 80°C rühren. Nach beendeter Reaktion (Nachweis beispielsweise über GC/MS) gibt man Wasser zur Reaktionsmischung und filtriert oder zentrifugiert vom ausgefallenen Palladiumschwarz ab, das anschließend recycelt werden kann. Das Produkt kann in an sich bekannter Weise beispielsweise durch Abdampfen von Lösungsmittel gewonnen werden und gegebenenfalls weiterhin zum Beispiel durch Destillation, Sublimation, Umkristallisation oder Umfällung weiter gereinigt werden.

In einer weiteren bevorzugten Ausführungsform legt man das Arylhalogenid oder das Arylsulfonat der allgemeinen Formel (III), die Arylboronsäure der allgemeinen Formel (IVa), den Komplex der allgemeinen Formel (VIII) und die Base unter Schutzgasatmosphäre vor, gibt dann Lösungsmittel zu und lässt bei einer Temperatur zwischen 20 und 80°C rühren, bis der Umsatz 95 % übersteigt.

Durch das erfindungsgemäße Verfahren werden in vorteilhafter Weise Polyarylverbindungen der allgemeinen Formel (XV) erhalten,

Ar¹-Ar² (XV)

in der Ar¹ und Ar² die unter den allgemeinen Formeln (III) und (IVa bis d) genannten Bedeutungen besitzen.

Die auf erfindungsgemäße Weise hergestellten Polyarylverbindungen eignen sich insbesondere zur Herstellung von Arzneimitteln, Agrochemikalien, und Polymeren, insbesondere leitfähigen Polymeren.

Der besondere Vorteil der vorliegenden Erfindung ist, dass durch die Bereitstellung neuer Salze und den davon abgeleiteten N-heterocyclischen Carbene und deren Komplexe neuartige Katalysatoren zur Verfügung stehen, die in überlegener Weise die Suzuki-Kupplung insbesondere von Arylchloriden mit reaktiven Arylverbindungen bereits bei Raumtemperatur mit sehr guten Ausbeuten und bislang unerreichten Aktivitäten ermöglichen.

### Beispiele

### Beispiel 1

### Herstellung von Bis (1,3-diadamantyl-2-yliden)palladium(0)

Bis-tri-tert.-butylphosphan)palladium(0) (1000 mg, 1.81 mmol) wurde in 30 mL *n-*Hexan gelöst. Eine Lösung vom 1,3-Diadamantylimidazol-2-yliden (1400 mg, 4.16 mmol) in 30 mL n-Hexan wurde zugegeben. Die Mischung wurde bei Raumtemperatur über 24 bis 48 h gerührt, wobei ein gelber Feststoff ausfiel. Nach Filtration und Trocknen *in vacuo* erhielt man einen hellgelben Feststoff. Die Röntgenstrukturanalyse wurde nach Umkristallisieren aus Diethylether durchgeführt.
Ausbeute: 1 177 mg, 1.51 mmol, 83 % der Theorie.

### Beispiel 2

### Alternative Darstellung über phosphanfreie Palladiumvorstufe

1,3-Diadamantylimidazol-2-yliden (222.1 mg, 0.66 mmol), Allylpalladium(II)-chloriddimer (60.4 mg, 0.165 mmol) und Natriumdimethylmalonat (50.9 mg, 0,33 mmol) werden unter Stickstoffatrnosphäre in 20 mL Toluol gelöst. Die Mischung wird iin einem Schlenkkolben für 16 h auf 90°C erhitzt. Danach wird von kleinen Mengen Palladiumschwarz und dem entstandenen NaCl abgefiltert, das Filtrat auf die Hälfte eingeengt und bei -78°C das Produkt ausgefällt. Ausbeute: 102 mg, 0.13 mmol, 40 % der Theorie.

Schmelzpunkt >285°C (Zersetzung); ¹H NMR (400 MHz, d8-Toluol, 25°C): δ = 1.64 (m, 24 H, CH₂C₁₀H₁₅), 180 (m, 12 H, CHC₁₀H₁₅), 2.11 (m, 24 H, CH₂C₁₀H₁₅), 6.69 (s, 4 H, NCHCHN); ¹³C{¹H} NMR (100.5 MHz, d8-toluene, 25°C); δ = 31.0, 36.8, 44.0 (C₁₀H₁₅), 57.2 (iso-C C₁₀H₁₅), 112.7 (NCHCHN), 191.8 (NCN); Cl-MS; m/z (%): 778 (3) [M⁺] 336 (100) [NHC⁺], 281 (33), 207 (27), 203 (40); C₄₆H₆₄N₄Pd (779.50): ber. C 70.88, H 8,22, N 7.19; gefunden C 70.80, H 8,24, N 7.22.

### Beispiel 3

### Kupplung von Arylhalogeniden mit Arylboronsäuren: Allgemeine Vorschrift

In einem Schlenkrohr wurden Bis(1,3-diadamantyl-2-yliden)palladium(0) (23,4 mg, 0,03 mmol), CsF (303,8 mg, 2 mmol), die Arylboronsäure (1,5 mmol) und das Arylchlorid (1 mmol) unter Stickstoffatmosphäre vorgelegt. Nach Zugabe von 3 mL 1,4-Dioxan wurde die Mischung bei Raumtemperaturgerührt. Die Reaktion wurde nach der angegebenen Zeit durch Zugabe von einigen Tropfen Wasser beendet und vom Palladiumschwarz abfiltriert. Die Ausbeute wurde über GC/MS Analyse mit Diethylenglycol-di-n-butylether als internen Standard bestimmt.

Die Ergebnisse der Katalysen sind in Tabelle 1 wiedergegeben.

**Tabelle 1**

| | **Aryl-X** | **Aryl-B(OH)**_{**2**} | **Zeit** | **Temperatur** | **Umsatz** |
|---|---|---|---|---|---|
| a) | p-Chlortoluol | Phenylboronsäure | 6 h | RT | > 95 % |
| | | | 20 min | 80°C | >95 % |
| b) | p-Chlortoluol | 3-Methoxyphenylboronsäure | 25 h | RT | 80 % |
| | | | 2 h | 80°C | 88 % |
| c) | 1-Chlor-4-trifluormethylbenzol | Phenylboronsäure | 2 h | RT | 95 % |
| d) | 1-Chlor-4-trifluormethylbenzol | 3-Methoxyphenylboronsäure | 25 h | RT | 73 % |
| | | | 2 h | | > 95 % |
| e) | 4-Acetyl-1-chlorbenzol | 3-Methoxyphenylboronsäure | 25 h | RT | 95 % |
| f) | 1-Chlor-4-methoxybenzol | Phenylboronsäure | 6 h | RT | > 95 % |

## Patentansprüche

1. Verfahren zur Herstellung von Polyarylverbindungen, **dadurch gekennzeichnet, dass**
- Arylhalogenide oder Arylsulfonate
- mit reaktiven Arylverbindungen
- in Gegenwart eines Katalysators, der zumindest einen Komplex eines Übergangsmetalls ausgewählt aus der Gruppe Palladium oder Platin enthält, der wiederum mindestens einen N-heterocyclischen Carbene der allgemeinen Formel (I) enthält, 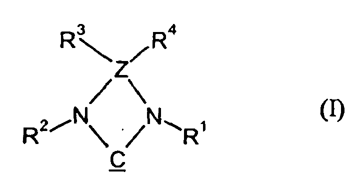 in der
Z für einen 1,2-Ethandiyl oder einen 1,2-Ethendiyl-Rest und
R¹ und R² jeweils unabhängig voneinander für Reste der allgemeinen Formel (II)
CR⁵ R⁶R⁷ (II)
stehen, in der
a) CR⁵R⁶R⁷ zusammen für einen substituierten oder unsubstituierten carbocyclischen oder heterocyclischen Rest oder einen substituierten oder unsubstituierten carbopolycyclischen oder heteropolycyclischen Rest steht oder
die Reste R⁵, R⁶ und R⁷ jeweils für Wasserstoff oder einen organischen Rest stehen,
wobei sowohl für a) als auch b) die Auflage gilt, dass von denjenigen drei Atomen von R⁵, R⁶ und R⁷, die an das Kohlenstoffatom C gebunden sind entweder
- alle drei jeweils unabhängig voneinander sekundäre, tertiäre oder quartäre Kohlenstoffatome sind oder
- zwei jeweils unabhängig voneinander sekundäre, tertiäre oder quartäre Kohlenstoffatome sind und für den Fall, dass beide dieser zwei Atome sekundär sind, zumindest eines davon an insgesamt mindestens zwei tertiäre oder quartäre Kohlenstoffatome gebunden ist und
R³ für Wasserstoff, Methyl, Benzyl und
R⁴ für Wasserstoff, C₁-C₈-Alkyl, Benzyl oder Phenyl steht,
miteinander umgesetzt werden.

2. Verfahren nach Anspruch 1, **dadurch gekennzeichnet, dass** es in Gegenwart von Base durchgeführt wird.

3. Verfahren nach Anspruch 2, **dadurch gekennzeichnet, dass** als Base Hydrogencarbonate, Carbonate, Methanolate, Ethanolate, Isopropylate, tert.-Butanolate und Acetate von Lithium, Natrium, Kalium und Cäsium sowie Cäsiumfluorid eingesetzt werden.

4. Verfahren nach einem oder mehreren der Ansprüche 2 bis 3, **dadurch gekennzeichnet, dass** als Base Cäsiumfluorid eingesetzt wird.

5. Verfahren nach einem oder mehreren der Ansprüche 1 bis 4, **dadurch gekennzeichnet, dass** es in Gegenwart von Lösungsmittel durchgeführt wird.

6. Verfahren nach einem oder mehreren der Ansprüche 1 bis 5, **dadurch gekennzeichnet, dass** als Arylhalogenide oder Arylsulfonate solche der allgemeinen Formel (III) eingesetzt werden,
Ar¹-Y (III)
in der
Ar¹ für einen substituierten oder unsubstituierten aromatischen Rest oder für einen substituierten oder unsubstituierten heteroaromatischen Rest und
Y für Chlor, Brom, Iod oder ein Sulfonat steht.

7. Verfahren nach einem oder mehreren der Ansprüche 1 bis 6, **dadurch gekennzeichnet, dass** als Arylhalogenide oder Arylsulfonate solche der allgemeinen Formel (III) eingesetzt werden, in der Ar¹ für einen substituierten oder unsubstituierten aromatischen Rest oder für einen substituierten oder unsubstituierten heteroaromatischen Rest und Y für Chlor steht.

8. Verfahren nach einem oder mehreren der Ansprüche 1 bis 7, **dadurch gekennzeichnet, dass** als reaktive Arylverbindungen solche der allgemeinen Formel (IVa, b, c und d) eingesetzt werden,
Ar²-B(OH)₂ (IVa)
Ar²-Sn(C₁-C₆-Alkyl)₃ (IVb)
Ar²-ZnX (IVc)
Ar²-MgX (IVd)
in der
Ar² für einen substituierten oder unsubstituierten aromatischen Rest oder für einen substituierten oder unsubstituierten heteroaromatischen Rest steht und X für Chlor, Brom oder Iod steht.

9. Verfahren nach einem oder mehreren der Ansprüche 1 bis 8, **dadurch gekennzeichnet, dass** als Katalysator Palladiumkomplexe der allgemeinen Formel (VIII) eingesetzt werden,
[Pd(L)₂] (VIII)
in der die beiden Liganden L identisch sind und für N-heterocyclische Carbene der allgemeinen Formel (I) stehen, in der die Reste Z, R¹, R², R³ und R⁴ die unter Anspruch 1 genannte Bedeutung besitzen.

10. Verfahren nach einem oder mehreren der Ansprüche 1 bis 9, **dadurch gekennzeichnet, dass** als Katalysator Palladiumkomplexe der allgemeinen Formel (VIII) eingesetzt werden, in der die beiden Liganden L identisch sind und für N-heterocyclische Carbene der allgemeinen Formel (I) stehen, in der Z für einen 1,2-Ethylendiyl-Rest und die Reste R¹ und R² identisch sind und die in Anspruch 1 genannte Bedeutung besitzen und R³ und R⁴ jeweils für Wasserstoff stehen.

11. Verfahren nach einem oder mehreren der Ansprüche 1 bis 10, **dadurch gekennzeichnet, dass** als Katalysator der Komplex [Bis-(1,3-Diadamantylimidazol-2-yliden)-palladium] eingesetzt wird.

12. Verfahren nach einem oder mehreren der Ansprüche 1 bis 11, **dadurch gekennzeichnet, dass** die Reaktionstemperatur 15 bis 40°C beträgt.

13. Palladium- und Platin-Komplexe, die als Liganden mindestens ein N-heterocyclisches Carben der allgemeinen Formel (I) enthalten, 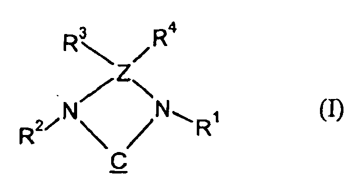 in der
Z für einen 1,2-Ethandiyl oder einen 1,2-Ethylendiyl-Rest und
R¹ und R² jeweils unabhängig voneinander für Reste der allgemeinen Formel (II)
CR⁵R⁶R⁷ (II)
stehen, in der
a) CR⁵R⁶R⁷ zusammen für einen substituierten oder unsubstituierten carbocyclischen oder heterocyclischen Rest oder einen substituierten oder unsubstituierten carbopolycyclischen oder heteropolycyclischen Rest steht oder
b) die Reste R⁵, R⁶ und R⁷ jeweils für Wasserstoff oder einen organischen Rest stehen,
wobei sowohl für a) als auch b) die Auflage gilt, dass von denjenigen drei Atomen von R⁵, R⁶ und R⁷, die an das Kohlenstoffatom C gebunden sind, entweder
- alle drei jeweils unabhängig voneinander sekundäre, tertiäre oder quartäre Kohlenstoffatome sind oder
- zwei jeweils unabhängig voneinander sekundäre, tertiäre oder quartäre Kohlenstoffatome sind und für den Fall, dass beide dieser zwei Atome sekundär sind, zumindest eines davon an insgesamt mindestens zwei tertiäre oder quartäre Kohlenstoffatome gebunden ist und
R³ und R⁴ jeweils unabhängig für Wasserstoff, C₆-C₁₂-Aryl, C₆-C₁₂-Arylalkyl oder C₁-C₈-Alkyl stehen.

14. [Bis-(1,3-Diadamantylimidazol-2-yliden)-palladium]

15. Verwendung von Verbindungen gemäß der Ansprüche 13 oder 14 als Katalysator.

16. Verfahren zur Herstellung von Komplexen der allgemeinen Formel (VIII),
[Pd(L)₂] (VIII)
in der die beiden Liganden L jeweils unabhängig voneinander für N-heterocyclische Carbene stehen **dadurch gekennzeichnet, dass**
Palladiurnkomplexe der allgemeinen Formel (IX),
[Pd(P)₂] (IX)
in der P für einen monodentaten Phosphanliganden steht mit einem N-heterocyclischen Carben in Gegenwart von Lösungsmittel umgesetzt werden.

## Claims

1. Process for preparing polyaryl compounds, **characterized in that**
- aryl halides or aryl sulphonates are reacted together
- with reactive aryl compounds
- in the presence of a catalyst which comprises at least one complex of a transition metal selected from the group of nickel, palladium or platinum, which in turn comprises at least one N-heterocyclic carbene of the general formula (I) 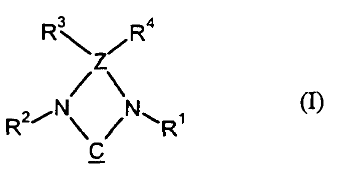
in which
Z is a 1,2-ethanediyl or 1,2-ethenediyl radical, and
R¹ and R² are each, independently of one another, radicals of the general formula (II)
CR⁵R⁶R⁷ (II)
in which
a) CR⁵R⁶R⁷ as a whole is a substituted or unsubstituted carbocyclic or heterocyclic radical or a substituted or unsubstituted carbopolycyclic or heteropolycyclic radical, or
the radicals R⁵, R⁶ and R⁷ are each hydrogen or an organic radical,
with the proviso both for a) and for b) that, of the three atoms of R⁵, R⁶ and R⁷ which are bonded to the carbon atom C, either
- all three are, in each case independently of one another, secondary, tertiary or quaternary carbon atoms, or
- two are, in each case independently of one another, secondary, tertiary or quaternary carbon atoms and, in the case where both of these two atoms are secondary, at least one thereof is bonded to a total of at least two tertiary or quaternary carbon atoms, and
R³ is hydrogen, methyl, benzyl and
R⁴ is hydrogen, C₁-C₈-alkyl, benzyl orphenyl.

2. Process according to Claim 1, **characterized in that** it is carried out in the presence of base.

3. Process according to Claim 2, **characterized in that** bicarbonates, carbonates, methanolates, ethanolates, isopropoxides, tert-butanolates and acetates of lithium, sodium, potassium and caesium, and caesium fluoride, are employed as base.

4. Process according to one or more of Claims 2 to 3, **characterized in that** caesium fluoride is employed as base.

5. Process according to one or more of Claims 1 to 4, **characterized in that** it is carried out in the presence of solvent.

6. Process according to one or more of Claims 1 to 5, **characterized in that** the aryl halides or aryl sulphonates employed are those of the general formula (III)
Ar¹-Y (III)
in which
Ar¹ is a substituted or unsubstituted aromatic radical or a substituted or unsubstituted heteroaromatic radical and
Y is chlorine, bromine, iodine or a sulphonate.

7. Process according to one or more of Claims 1 to 6, **characterized in that** the aryl halides or aryl sulphonates employed are those of the general formula (III) in which Ar¹ is a substituted or unsubstituted aromatic radical or a substituted or unsubstituted heteroaromatic radical and Y is chlorine.

8. Process according to one or more of Claims 1 to 7, **characterized in that** the reactive aryl compounds employed are those of the general formula (IVa, b, c and d)
Ar²-B(OH)₂ (IVa)
Ar²-Sn(C₁-C₆-alkyl)₃ (IVb)
Ar²-ZnX (IVc)
Ar²-MgX (IVd)
in which
Ar² is a substituted or unsubstituted aromatic radical or a substituted or unsubstituted heteroaromatic radical, and X is chlorine, bromine or iodine.

9. Process according to one or more of Claims 1 to 8, **characterized in that** palladium complexes of the general formula (VIII)
[Pd(L)₂] (VIII)
in which the two ligands L are identical and are N-heterocyclic carbenes of the general formula (I) in which the radicals Z, R¹, R², R³ and R⁴ have the meaning specified in Claim 1, are employed as catalyst.

10. Process according to one or more of Claims 1 to 9, **characterized in that** palladium complexes of the general formula (VIII) in which the two ligands L are identical and are N-heterocyclic carbenes of the general formula (I) in which Z is a 1,2-ethylenediyl radical and the radicals R¹ and R² are identical and have the meaning specified in Claim 1, and R³ and R⁴ are each hydrogen, are employed as catalyst.

11. Process according to one or more of Claims 1 to 10, **characterized in that** the complex [bis(1,3-diadamantylimidazol-2-ylidene)palladium] is employed as catalyst.

12. Process according to one or more of Claims I to 11, **characterized in that** the reaction temperature is 15 to 40°C.

13. Palladium and platinum complexes which comprise as ligands at least one N-heterocyclic carbene of the general formula (I) 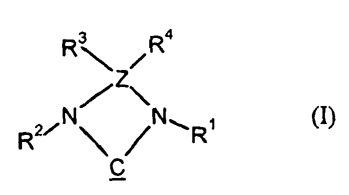 in which
Z is a 1,2-ethanediyl or a 1,2-ethylenediyl radical and
R¹ and R² are each, independently of one another, radicals of the general formula (II)
CR⁵R⁶R⁷ (II)
in which
a) CR⁵R⁶R⁷ as a whole is a substituted or unsubstituted carbocyclic or heterocyclic radical or a substituted or unsubstituted carbopolycyclic or heteropolycyclic radical, or
b) the radicals R⁵, R⁶ and R⁷ are each hydrogen or an organic radical,
with the proviso both for a) and for b) that, of the three atoms of R⁵, R⁶ and R⁷ which are bonded to the carbon atom C, either
- all three are, in each case independently of one another, secondary, tertiary or quaternary carbon atoms, or
- two are, in each case independently of one another, secondary, tertiary or quaternary carbon atoms and, in the case where both of these two atoms are secondary, at least one thereof is bonded to a total of at least two tertiary or quaternary carbon atoms, and
R³ and R⁴ are each independently hydrogen, C₆-C₁₂-aryl, C₆-C₁₂₋arylalkyl or C₁-C₈-alkyl.

14. [Bis(1,3-diadamantylimidazol-2-ylidene)palladium]

15. Use of compounds according to Claims 13 or 14 as catalyst.

16. Process for preparing complexes of the general formula (VIII)
[Pd(L)₂] (VIII)
in which the two ligands L are each, independently of one another, N-heterocyclic carbenes, **characterized in that**
palladium complexes of the general formula (IX)
[Pd(P)₂] (IX)
in which P is a monodentate phosphane ligand are reacted with an N-heterocyclic carbene in the presence of solvent.

## Revendications

1. Procédé de préparation de composés polyaryliques, **caractérisé en ce que** l'on fait réagir
- des halogénures d'aryle ou des sulfonates d'aryle
- avec des composés aryliques réactifs
- en présence d'un catalyseur, qui comprend au moins un complexe d'un métal de transition choisi parmi le groupe du palladium ou du platine, qui comprend à son tour un carbène N-hétérocyclique de formule générale (I), 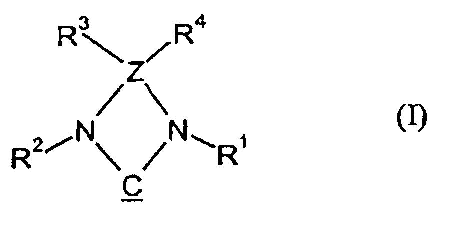
dans laquelle
Z représente un radical 1,2-éthanediyle ou 1,2-éthénediyle, et
R¹ et R² représentent chacun, indépendamment l'un R¹ de l'autre, des radicaux de formule générale (II)
CR⁵R⁶R⁷ (II)
dans laquelle
a) CR⁵R⁶R⁷ représente conjointement un radical carbocyclique ou hétérocyclique substitué ou non substitué ou un radical carbopolycyclique ou hétéropolycyclique substitué ou non substitué, ou
b) les radicaux R⁵, R⁶ et R⁷ représentent chacun un hydrogène ou un radical organique,
à condition, aussi bien pour a) que pour b), que parmi les trois atomes de R⁵, R⁶ et R⁷ qui sont liés à l'atome de carbone C, soit
- tous les trois, dans chaque cas indépendamment les uns des autres, sont des atomes de carbone secondaires, tertiaires ou quaternaires, soit
- deux, dans chaque cas indépendamment l'un de l'autre, sont des atomes de carbone secondaires, tertiaires ou quaternaires, et au cas où les deux de ces deux atomes sont secondaires, au moins l'un d'entre eux est lié à un total d'au moins deux atomes de carbone tertiaires ou quaternaires, et
R³ représente un hydrogène, un méthyle, un benzyle, et
R⁴ représente un hydrogène, un alkyle en C₁-C₈, un benzyle ou un phényle.

2. Procédé selon la revendication 1, **caractérisé en ce qu'**il est réalisé en présence d'une base.

3. Procédé selon la revendication 2, **caractérisé en ce que** l'on utilise, en tant que base, des hydrogénocarbonates, des carbonates, des méthanolates, des éthanolates, des isopropylates, des tert-butanolate et des acétates de lithium, de sodium, de potassium et de césium ainsi que le fluorure de césium.

4. Procédé selon l'une ou plusieurs des revendications 2 à 3, **caractérisé en ce que** l'on utilise le fluorure de césium en tant que base.

5. Procédé selon l'une ou plusieurs des revendications 1 à 4, **caractérisé en ce qu'**il est réalisé en présence d'un solvant.

6. Procédé selon l'une ou plusieurs des revendications 1 à 5, **caractérisé en ce que** l'on utilise, en tant qu'halogénures d'aryle ou sulfonates d'aryle, ceux de formule générale (III),
Ar¹-Y (III)
dans laquelle
Ar¹ représente un radical aromatique substitué ou non substitué ou un radical hétéroaromatique substitué ou non substitué, et
Y représente un chlore, un brome, un iode ou un sulfonate.

7. Procédé selon l'une ou plusieurs des revendications 1 à 6, **caractérisé en ce que** l'on utilise, en tant qu'halogénures d'aryle ou sulfonates d'aryle, ceux de formule générale (III) dans laquelle Ar¹ représente un radical aromatique substitué ou non substitué ou un radical hétéroaromatique substitué ou non substitué, et Y représente un chlore.

8. Procédé selon l'une ou plusieurs des revendications 1 à 7, **caractérisé en ce que** l'on utilise, en tant que composés aryliques réactifs, ceux de formules (IVa, b, c, et d),
Ar²-B(OH)₂ (IVa)
Ar²-Sn(C₁-C₆-alkyl)₃ (IVb)
Ar²-ZnX (IVc)
Ar²-MgX (IVd)
dans lesquelles
Ar² représente un radical aromatique substitué ou non substitué ou un radical hétéroaromatique substitué ou non substitué, et X représente un chlore, un brome ou un iode.

9. Procédé selon l'une ou plusieurs des revendications 1 à 8, **caractérisé en ce que** l'on utilise, en tant que catalyseur, des complexes du palladium de formule générale (VIII),
[Pd(L)₂] (VIII)
dans laquelle les deux ligands L sont identiques et représentent des carbènes N-hétérocycliques de formule (I), dans laquelle les radicaux Z, R¹, R², R³ et R⁴ présentent la signification mentionnée dans la revendication 1.

10. Procédé selon l'une ou plusieurs des revendications 1 à 9, **caractérisé en ce que** l'on utilise, en tant que catalyseur, des complexes du palladium de formule générale (VIII), dans laquelle les deux ligands L sont identiques et représentent des carbènes N-hétérocycliques de formule générale (I), dans laquelle z représente un radical 1,2-éthylènediyle et les radicaux R¹ et R² sont identiques et présentent la signification mentionnée dans la revendication 1, et R³ et R⁴ représentent chacun un hydrogène.

11. Procédé selon l'une ou plusieurs des revendications 1 à 10, **caractérisé en ce que** l'on utilise, en tant que catalyseur, le complexe [bis-(1,3-diadamantylimidazol-2-ylidène)-palladium].

12. Procédé selon l'une ou plusieurs des revendications 1 à 11, **caractérisé en ce que** la température de réaction est de 15 à 40ºC.

13. Complexe du palladium et du platine comprenant, en tant que ligands, au moins un carbène N-hétérocyclique de formule générale (I) 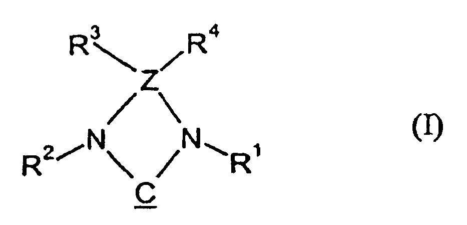 dans laquelle
Z représente un radical 1,2-éthanediyle ou 1,2-éthylènediyle, et
R¹ et R² représentent chacun, indépendamment l'un de l'autre, des radicaux de formule générale (II)
CR⁵R⁶R⁷ (II)
dans laquelle
a) CR⁵R⁶R⁷ représentent conjointement un radical carbocyclique ou hétérocyclique substitué ou non substitué ou un radical carbopolycyclique ou hétéropolycyclique substitué ou non substitué, ou
b) les radicaux R⁵, R⁶ et R⁷ représentent chacun un hydrogène ou un radical organique,
à condition, aussi bien pour a) que pour b), que parmi les trois atomes de R⁵, R⁶ et R⁷ qui sont liés à l'atome de carbone C, soit
- tous les trois, dans chaque cas indépendamment les uns des autres, sont des atomes de carbone secondaires, tertiaires ou quaternaires, soit
- deux, dans chaque cas indépendamment l'un de l'autre, sont des atomes de carbone secondaires, tertiaires ou quaternaires, et au cas où les deux de ces deux atomes sont secondaires, au moins l'un d'entre eux est lié à un total d'au moins deux atomes de carbone tertiaires ou quaternaires, et
R³ et R⁴ représentent chacun indépendamment un hydrogène, un aryle en C₆-C₁₂, un C₆-C₁₂₋arylalkyle ou un alkyle en C₁-C₈.

14. [Bis-(1,3-diadamantylimidazol-2-ylidène)-palladium]

15. Utilisation de composés selon la revendication 13 ou 14 en tant que catalyseur.

16. Procédé de préparation de complexes de formule (VIII),
[Pd(L)₂] (VIII)
dans laquelle les deux ligands L représentent chacun, indépendamment l'un de l'autre, des carbènes N-hétérocycliques, **caractérisé en ce que** l'on fait réagir
des complexes du palladium de formule générale (IX)
[Pd(P)₂] (IX)
dans laquelle P représente un ligand phosphane monodenté, avec un carbène N-hétérocyclique en présence d'un solvant.
